(19) Europäisches Patentamt / European Patent Office / Office européen des brevets

(11) **EP 1 811 863 B1**

(12) **EUROPEAN PATENT SPECIFICATION**

(45) Date of publication and mention
of the grant of the patent:
**12.09.2012 Bulletin 2012/37**

(51) Int Cl.:
*A23L 1/236* (2006.01)        *C12P 19/14* (2006.01)
*C08B 30/18* (2006.01)        *C09J 103/02* (2006.01)

(21) Application number: **05816044.1**

(22) Date of filing: **21.10.2005**

(86) International application number:
**PCT/US2005/037592**

(87) International publication number:
**WO 2006/047176 (04.05.2006 Gazette 2006/18)**

(54) **PROCESS FOR THE PRODUCTION OF MALTODEXTRINS**

VERFAHREN ZUR HERSTELLUNG VON MALTODEXTRINEN

PROCEDE DE PRODUCTION DE MALTODEXTRINE

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.10.2004  US 621484 P**
          **12.08.2005  US 707915 P**

(43) Date of publication of application:
**01.08.2007  Bulletin 2007/31**

(60) Divisional application:
**11169546.6 / 2 368 443**

(73) Proprietor: **CARGILL, INCORPORATED**
**Wayzata, Minnesota 55391 (US)**

(72) Inventors:
  • **DELEYN, Francois**
    **B-9860 Oosterzele (BE)**
  • **AIKEN, Christopher**
    **Valparaiso, Indiana 46383 (US)**
  • **DEREZ, Frank**
    **B-1600 Sint-Pieters-Leeuw (BE)**
  • **MAURO, David**
    **Dolton, Illinois 60419 (US)**
  • **PROVOOST, Dirk**
    **B-1800 Vilvoorde (BE)**
  • **STALIN, Marie-Odile**
    **F-59000 Lille (FR)**
  • **VANHEMELRIJCK, Bruno**
    **B-1731 Asse (BE)**

(74) Representative: **Gowshall, Jonathan Vallance et al**
**Forresters**
**Skygarden**
**Erika-Mann-Strasse 11**
**80636 München (DE)**

(56) References cited:
**US-A- 3 849 194      US-A- 3 853 706**
**US-A- 3 974 034      US-A- 4 062 728**
**US-A- 4 410 368**

Remarks:
The file contains technical information submitted after the application was filed and not included in this specification

**Description**

FIELD OF THE INVENTION

The present invention relates to a process for preparing maltodextrins. The invention also relates to certain maltodextrins.

BACKGROUND

Maltodextrins are generally known in the art. Maltodextrins may be produced by the hydrolysis of starch with either acids or enzymes. Exemplary patents are U.S. Patent Numbers 3,849,194; 3,853,706; 4,284,722; 4,447,532 and 5,612,202. Maltodextrins are characterized by DE value, expressing the level of starch conversion. DE is an abbreviation for Dextrose Equivalent, a common expression in the art for describing the total reducing sugars content of a material, expressed as percent dextrose, dry basis. Several objectives in the preparation of commercial maltodextrins are to attain stability, clarity and non-crystallizing characteristics. Maltodextrins are useful for a variety of food applications due to their bland taste, low sweetness and low hygroscopicity. For example, they are useful as bulking agents, carriers, flavor enhancers, moisture-holding agents, dispersing agents, film-forming agents, encapsulating agents and the like.
Various maltodextrins are available commercially. There is a continuing need, however, for processes for producing maltodextrins that are easier and simpler to carry out, and for maltodextrins that possess good clarity and/or low turbidity over a period of time.

DETAILED DESCRIPTION OF THE DISCLOSURE

The invention is defined in the claims.
The present disclosure relates to a process for preparing maltodextrins and certain maltodextrins. This process comprises mixing starch with water to form a starch slurry of less than about 50% dry substance (hereinafter "ds"). In another embodiment, the starch slurry has about 24% ds to about 40% ds, or in yet another embodiment about 32% ds to about 36% ds. The starch may be derived from any starch source, such as cereal starches and root starches. Typical of these starches are dent corn, waxy corn, potato, wheat, rice, sago, tapioca, sorghum, sweet potato, or mixtures thereof. The starch slurry may be supplemented with an aqueous calcium-containing solution such as calcium chloride solution to provide 50-100 ppm free calcium in the starch slurry. The starch slurry may be heated below the gelatinization temperature of the starch in the starch slurry preparation tank. The starch slurry is contacted with a sufficient amount of a *Bacillus stearothermophilus* alpha-amylase to convert or hydrolyze the starch. Suitable *Bacillus stearothermophilus* alpha-amylases include GEN-ZYME G995, manufactured and sold by Genencor International, Palo Alto, California, and TERMAMYL 120L Type: S, manufactured and sold by Novozymes A/S, Denmark. For example, the *Bacillus stearothermophilus,* alpha-amylase may be used in an amount ranging from about 0.01% to about 0.09% by weight of the starch on dry basis.
The pH of the enzyme-containing starch slurry is selected to provide a suitable activity of *Bacillus stearothermophilus* alpha-amylase. Generally, the pH ranges from about 5.0 to about 7.0, or from about 5.7 to about 6.3 in another embodiment, or from about 5.9 to about 6.1 in yet another embodiment. The pH as described herein is maintained throughout the entire process, except in the saccharification step where the pH is reduced to inactivate the enzyme after the desired DE has been obtained. The enzyme-containing starch slurry is heated to a temperature of about 80° C. to about 115° C. in one embodiment, in another embodiment from about 102° C. to about 115° C., or from about 107° C. to about 110° C. in another embodiment, for about 6 to about 15 minutes to form a first liquefact.
The first liquefact is optionally cooled. In one embodiment, cooling is achieved by flash cooling wherein, the pressure is released quickly to atmospheric level and the temperature is dropped rapidly to a temperature from about 93° C. to about 100° C. By "quickly" is meant the pressure is released within about 1 to about 5 seconds and by "rapidly" is meant the temperature is dropped within about 1 to about 5 seconds. The resultant product has a DE of about 0.5 to about 5.0, or of about 1.0 to about 3.0 DE in another embodiment. The temperature of the first liquefact is then adjusted to from about 120°C. to about 165°C. in one embodiment, in another embodiment from about 130° C. to about 165° C., or from about 150° C. to about 165° C. in another embodiment, or from about 158° C. to about 165° C. in yet another embodiment, and maintained at this temperature for about 30 seconds to about 10 minutes. In another embodiment, the residence time is from about 1 minute to about 6 minutes, or from about 3 minutes to about 5 minutes in yet another embodiment.
Subsequently, the temperature of the first liquefact is reduced to a temperature from about 101° C. to about 115° C., or from about 108° C. to about 110° C. in another embodiment, for up to about 15 minutes, preferably about 2 to about 15 minutes. In another embodiment, the residence time is from about 3 minutes to about 8 minutes, or from about 3 minutes to about 5 minutes in yet another embodiment. This temperature reduction is performed in a pressure vessel. To the first liquefact is added a second dose of *Bacillus stearothermophilus* alpha-amylase either prior to introducing the first liquefact into a pressure vessel or directly into the pressure vessel. The amount of the second dose of *Bacillus stearothermophilus* alpha-amylase is sufficient to produce a maltodextrin product having a DE of from about 5 to less than about 20. For example, the amount of the second dose of *Bacillus stearothermophilus* alpha-amylase may be used in an amount ranging from about 0.01% to about 0.09% by weight of the starch on dry basis. The resultant liquefact after contact with the second dose of *Bacillus stearothermophilus* alpha-amylase is hereinafter referred to as "second liquefact".

The second liquefact is cooled to a temperature from about 93° C. to about 100° C., which is maintained for about 2 minutes to about 15 minutes. In one embodiment, the cooling is achieved by flash cooling. In another embodiment, the residence time is from about 3 minutes to about 10 minutes, or from about 3 minutes to about 4 minutes in yet another embodiment. The temperature of the second liquefact is then maintained at about 93°C. to about 100° C., or in another embodiment to about 93° C. to about 98° C., in a saccharification tank, holding pipe or the equivalent, for a period of time until a maltodextrin product having a D.E. of about 5 to less than about 20 is produced. Thereafter, the pH is adjusted to about 3.4 to about 3.7 to inactivate the hydrolytic action of the *Bacillus stearothermophilus* alpha-amylase.

The processing conditions may vary within certain limits dictated by the stability and activity characteristics of the enzyme and the gelatinization properties of the starch. For example, the quantity of *Bacillus stearothermophilus* alpha-amylase required for obtaining a maltodextrin with the desired DE will depend upon the activity of the *Bacillus stearothermophilus* alpha-amylase, the temperature, the DE after the first liquefaction, the pH of the first and second liquefacts, and the desired final DE.

The resulting maltodextrin product is in liquid form. The liquid maltodextrin product may be concentrated to yield a syrup having any desired solids content such as, for example, greater than 50% ds. Moreover, the liquid maltodextrin product may be spray dried if desired to a powder.

The liquid maltodextrin of the present process is refined by conventional refining methods. For example, the refining methods include filtration through diatomaceous earth on a fixed or rotary vacuum filter, centrifugation, flocculation, flotation and the like, and treatment with vegetable carbon and ion exchange resins. Furthermore, the final refined liquid maltodextrin product optionally can be spray dried to a powder.

The present disclosure also relates to certain novel liquid maltodextrins. The novel liquid maltodextrins may be produced by the process described herein.

The novel liquid maltodextrins are characterized by having a DE value ranging, from about 9 to about 15, and in another embodiment a value of about 10 to about 13, and in yet a further embodiment, a value of from about 9 to about 10.5. Further, the novel liquid maltodextrins are characterized by having a value for percent light transmittance at 390 nm, of at least 30%, at a ds of about 62% to about 67%, after a period of at least 28 days. In another embodiment, the liquid maltodextrins have a value for percent light transmittance of at least about 40%, and in yet a further embodiment, have a percent light transmittance of at least about 79%.

The maltodextrins of the present disclosure, whether in the form of syrups or dry powder, are generally characterized by blandness of taste and low sweetness. When used in food products, the maltodextrins generally have a minimal effect upon flavor while providing bulk, stability, favorable mouthfeel characteristics and increased nutritive value.

These characteristics make the products of the present disclosure generally suitable for applications as carriers for coloring agents, flavors, essences and synthetic sweeteners; spray drying adjuncts for coffee extracts and tea extracts, bulking, boding and dispersing agents in synthetic creams or coffee whiteners; ingredients promoting a moisture retention in bread, pastry and meats; components of dry soup mixes, bakery mixes, frosting mixes, spice mixes and blends, beverage powders, condiments, gravy mixes, sauce mixes and frozen dairy foods; and in fat mimetics. In addition, they are generally useful in the formulation of tabletting compounds which can be used in food products or pharmaceutical products, anti-caking agents, whipped products, protective coatings, agglomeration aids, low or reduced-in-calorie foods and beverages, and low or reduced-in-fat foods and beverages.

## EXAMPLES

In carrying out the examples, the following procedures were used to test the refined liquid maltodextrins prepared in accordance with this disclosure.

DE:

DE is measured according to the Lane-Eynon method, which is commonly used in the industry to measure dextrose equivalent (Official Methods of Analysis (1990), Association of Official Analytical Chemists, 15th Edition, Method 923.09, "Invert Sugar in Sugars and Syrups, Lane-Eynon General Volumetric Method, Final Action," p. 1016).

Clarity - Test Procedure A

Clarity of the refined liquid maltodextrin products is determined by measuring the amount of light passing through a test sample as compared to that passing through a blank of distilled water. The test samples were examined spectrophotometrically by measuring the percent light transmittance at 600 nm through 4 cm cells, each containing portions of the test samples, which were concentrated to 65% ds. A Shimadzu UV1650 spectrophotometer (available from Shimadzu Deutschland GmB H, Duisburg, Germany) was used to measure the clarity of the test samples. The test samples stored at 5° C. were measured over a period of time to determine whether the clarity was stable.

Clarity -Test Procedure B

Clarity of the refined liquid maltodextrin products is determined by measuring the amount of light passing through a test sample as compared to that passing through a blank of distilled water. The test samples were examined spectrophotometrically by measuring the percent light transmittance at 390 nm through 4 cm cells, each containing portions of the test samples, which were concentrated to about 62 to about 67% ds. A Spectronic Model Genesys 5 spectrophotometer was used to measure the clarity of the test samples. The test samples stored at 54.4°C (130°F) were measured over a period of time to determine whether the clarity was stable.

Turbidity:

Turbidity of the test samples at 30% ds and 65% ds, is measured in comparison with turbidity standards using a HACH Laboratory Turbidimeter Type 2100N (available from Hach Company, Loveland, Colorado) and expressed in NTU, turbidity units. The procedure used to measure turbidity is the procedure described in the instruction manual provided by the Hach Company. The lower the turbidity, the higher the clarity. The test samples stored at 5° C., 20° C., 25° C. and 60° C. were measured over a period of time to determine whether the turbidity was stable.

Molecular Weight Distribution:

The molecular weight distribution of the refined liquid maltodextrin products is measured by the degree of polymerization (DP). DP is the average number of anhydroglucose units in the maltodextrin molecule. The molecular weight distribution is assayed by gel permeation chromatography of an aqueous solution of the maltodextrin (about 10 % ds). The sample is chromatographed on a Waters Chromatograph equipped with two columns in series (Shodex S-803 and Shodex S-801 from Showa Denko, Japan), at 70°C, eluted with HPLC grade water at a flow of 1 ml/min. Detection is done by a differential refractometer. Polymer reference products (low polydispersivity pullulans from Showa Denko, Japan) are used to relate elution time to the molecular weight of the different fraction of the assayed product.

Number Average Molecular Weight (Mn)

Mn was calculated using the following equation:

$$\overline{M}_n = \frac{\Sigma\, N_i\, M_i}{\Sigma\, N_i}$$

Where $N_i$ is the number of moles having a molecular weight of $M_i$
Reference: Application Note AN 232-10, Dale R. Baker, Hewlett-Packard Co, Avondale PA.
Weight Average Molecular Weight (Mw)
Mw was calculated using the following equation:

$$\overline{M}_w = \frac{\Sigma\, N_i\, M_i^2}{\Sigma\, N_i M_i}$$

Reference: Application Note AN 232-10, Dale R. Baker, Hew lett-Packard Co, Avondale PA.

EXAMPLE 1

Dent corn starch was mixed with water to produce a 32 % to 34% ds starch slurry and the pH of the starch slurry was adjusted to 5.7 to 6.3 with 10% soda ash. To this starch slurry were added 50 to 70 ppm $Ca^{++}$ (calcium chloride) and GEN-ZYME G995 *Bacillus stearothermophilus* alpha-amylase at 0.02 % ds starch. The enzyme-containing, starch slurry was pumped at a flow rate of about 150 liters/hour to a series of holding tubes where steam (at feed pressure of 1000000pa to 1100000pa (10-11 bars)) was injected and a back-pressure of 60000pa to 80000pa (0.6-0-0.8 bar) was applied to raise the temperature to about 108°C. The enzyme-containing starch slurry was held at this temperature for about 9 minutes to form a first liquefact and then flash cooled to atmospheric pressure to thereby reduce the temperature to about 98°C. At this point, the DE of the first liquefact was from about 1 to about 3. The first liquefact was pumped to another holding tube where steam (feed pressure of 1000000pa to 1100000pa (10-11 bars)) was injected and a back-pressure of 600000pa to 650000pa (6.0-6.5 bars) was applied to raise the temperature to about 160°C. The first liquefact was held at this temperature for about 3 minutes. A second dose of GEN-ZYME G995 Bacillus stearothermophilus alpha-amylase at 0.02% ds starch was added to the first liquefact prior to being pumped into an 8-liter pressure vessel where the first liquefact had been held for about 3 minutes at a temperature of about 107°C by applying a back pressure of 39000pa to 40000pa (0.39-0.40 bar). A second liquefact was thereby formed. The second liquefact was then flash cooled to atmospheric pressure to thereby reduce the temperature to about 95 ° C. The second liquefact was collected in a saccharification tank and allowed to convert for a period of about 5.0 hours to yield a liquid maltodextrin product with a DE of about 13.9. Throughout the process, the flow rate was maintained at about 150 liter/hour and the pH was maintained from about 5.7 to about 6.3. Thereafter, a sufficient amount of 32% hydrochloric acid was added to lower the pH to about 3.5 to inactivate any residual enzyme.

EXAMPLE 2

The liquid maltodextrin product of Example 1 was then refined by the following conventional refining method. The product was filtered at 80° C. to remove insoluble materials, such as fat and protein, using a NIVOBA® rotary vacuum filter (available from Nivoba B.V., Groningen, Netherlands) with a CELITE 555 filter (available from Celite Corporation, Santa Barbara, California). The temperature was lowered to 65° C. and the product was decolorized using 500 ml Lurgi's Epilon MC-h 1240 granular carbon. Minerals were removed by using ion exchange resins (DOW 88 Mono cation exchange resin (80 ml); DOW 66 Mono anion exchange resin (100 ml); and Mitsubishi Relite RAD/F polishing resin (50 ml)). The liquid maltodextrin was concentrated to 30% ds and 65% ds. The analysis of the refined liquid maltodextrin product is given in Tables 1 and 2.

TABLE 1

| ANALYSIS OF MALTODEXTRIN OF EXAMPLE 2 | |
|---|---|
| DE | 13.9 |
| pH | 4-5 |
| Molecular Weight Distribution (DP) | % |
| 1-5 | 15.3 |
| 6-9 | 20.1 |
| 10-19 | 11.8 |
| 20-45 | 11.3 |
| 46-125 | 13.8 |
| 126-280 | 11.9 |
| 281-600 | 9.1 |
| 601-1500 | 4.1 |
| >1500 | 2.6 |
| Total | 100.0 |
| Mn | 1714 |
| Mw | 32,439 |

## TABLE 2

| STABILITY RESULTS | | | | | | | |
|---|---|---|---|---|---|---|---|
| | | TURBIDITY (NTU) | | | | | Clarity 600 nm % |
| % ds | | 30 | 30 | 30 | 65 | 65 | 65 | 65 |
| Temperature °C | | 25 | 60 | 5 | 25 | 60 | 5 | 5 |
| Stability time Hours | 0 | 1.8 | 1.8 | 1.9 | 0.6 | 0.7 | 4.7 | 100.0 |
| | 24 | 1.8 | 1.8 | 1.9 | 1.3 | 1.1 | 1.7 | 100.0 |
| | 48 | 1.8 | 1.9 | 1.9 | 56.0 | 3.0 | 9.5 | 94.8 |
| | 120 | 1.8 | 2.0 | 1.9 | 1733.0 | 10.5 | 67.8 | 4.8 |
| | 312 | 1.8 | 2.1 | 1.9 | - | - | - | - |

The resulting refined liquid maltodextrin products at 30% ds are very stable at 5° C., 25° C., and 60° C. Generally, the clarity of such products is stable for up to 13 or more days.

From the above data, it is apparent that the clarity of the refined liquid maltodextrin product stored at 5° C. at 65% ds for up to about 48 hours is good as indicated by the high light transmittance values of 100% light transmittance at 24 hours and 94.8% light transmittance at 48 hours as compared to the control of 100% light transmittance at 0 hours. The turbidity of the refined liquid maltodextrin product stored at 5° C., 25° C. and 60° C., at 30% ds is about equivalent to the control at 0 hours for up to 13 days of storage, which is indicative of a stable product. Similar results are shown for turbidity at 65% ds for a period of up to about 24 hours.

EXAMPLE 3

Dent corn starch was mixed with water to produce a 32% to 34% ds starch slurry and the pH was adjusted to 5.7 to 6.3 with 10% soda ash. To this starch slurry were added 50 to 70 ppm $Ce^{++}$ (calcium chloride) and TERMAMYL 120L Type *S Bacillus stearothermophilus alpha-amylase at* 0.035 % ds starch. The enzyme-containing starch slurry was pumped at a flow rate of about 150 liter/hour to a series of holding tubes where steam (at feed pressure of 700000pa to 800000pa (7-8 bars)) was injected and a back pressure of 60000pa to 80000pa (0.6-0.8 bar) was applied to raise the temperature to about 108°C. The enzyme-containing starch slurry was held at this temperature for about 9 minutes to form a first liquefact and then flash cooled to atmospheric pressure to thereby reduce the temperature to about 98°C. At this point, the DE of the first liquefact was from about 1 to about 3. The first liquefact was pumped to another holding tube where steam (feed pressure of 1000000pa to 1100000pa (10-11 bars) was injected and a back pressure of 600000pa to 650000pa (6.0-6.5 bars) was applied to raise the temperature to about 160°C. The first liquefact was held at this temperature for about 3 minutes. A second dose of TERMAMYL 120L Type S *Bacillus stearothermophilus* alpha-amylase at 0.01% ds starch was added to the first liquefact prior to being pumped into an 8-liter pressure vessel where the first liquefact had been held for about 3 minutes at a temperature of about 107°C by applying a back pressure of 34000pa to 40000pa (0.39-0.40 bar). A second liquefact was thereby formed. The second liquefact was then flash cooled to atmospheric pressure to thereby reduce the temperature to about 95°C. The second liquefact was collected in a saccharification tank and allowed to convert further for a period of about 8 hours to yield a liquid maltodextrin product with a DE of about 13.1. Thereafter, a sufficient amount of 32% hydrochloric acid was added to lower the pH to about 3.5 to inactivate any residual enzyme. Throughout the process, the flow rate was maintained at about 150 liter/hour and the pH was maintained from about 5.7 to about 6.3, except at the end of the saccharification where the pH was reduced to 3.5 to inactivate the enzyme.

EXAMPLE 4

The liquid maltodextrin product of Example 3 was then refined and concentrated as described in Example 2. The analysis of the refined liquid maltodextrin product is given in Tables 3 and 4.

TABLE 3

| ANALYSIS OF MALTODEXTRIN OF EXAMPLE 4 | |
| --- | --- |
| Dry solids | 30% |
| DE | 13.1 |
| pH | 4-5 |
| Molecular Weight Distribution (DP) | % |
| 1-5 | 18.4 |
| 6-9 | 22.5 |
| 10-19 | 10.3 |
| 20-45 | 9.4 |
| 46-125 | 11.4 |
| 126-280 | 10.7 |
| 281-600 | 8.8 |
| 601-1500 | 5.2 |
| >1500 | 3.5 |
| Total | 100.0 |
| Mn | 1521 |
| Mw | 38,112 |

TABLE 4

| TURBIDITY OF 13.1 DE MALTODEXTRIN OF EXAMPLE 4 | |
| --- | --- |
| Storage Time at 20° C. (hours) | Turbidity (NTU) |
| O | 1.8 |
| 24 | 1.8 |
| 62 | 2.0 |

The 13.1 DE refined liquid maltodextrin product prepared with TERMAMYL 120L Type S *Bacillus stearothermophilus* alpha-amylase showed very low turbidity up to 62 hours of storage at 20° C., which is indicative of a clear and stable product.

EXAMPLE 5

The process of Example 3 was followed, except a 18.6 DE liquid maltodextrin was prepared using TERMAMYL 120L Type S alpha-amylase at 0.035% ds as the first dose and 0.01 % ds of the same alpha-amylase as the second dose. The total time for saccharification was about 24 hours and a maltodextrin product with a DE of about 18.6 was obtained. The maltodextrin product was refined in the same way as described in Example 2 and concentrated to 30% ds. The analysis of the refined liquid maltodextrin product is given in Tables 5 and 6.

TABLE 5

| ANALYSIS OF MALTODEXTRIN OF EXAMPLE 5 | |
| --- | --- |
| Dry solids | 30% |
| DE | 18.6 |
| pH | 4-5 |
| Molecular Weight Distribution (DP) | % |
| 1-5 | 26.9 |

(continued)

| Molecular Weight Distribution (DP) | % |
|---|---|
| 6-9 | 29.0 |
| 10-19 | 6.2 |
| 20-45 | 9.3 |
| 46-125 | 11.5 |
| 126-280 | 8.3 |
| 281-600 | 5.2 |
| 601-1500 | 2.3 |
| >1500 | 1.2 |
| Total | 100.0 |
| Mn | 1,145 |
| Mw | 19,424 |

TABLE 6

| TURBIDITY OF 18.6 DE MALTODEXTRIN OF EXAMPLE 5 | |
|---|---|
| | |
| Storage Time at 20° C. (hours) | Turbidity (NTU) |
| 0 | 1.5 |
| 24 | 1.5 |
| 71 | 1.9 |

The 18.6 DE refined liquid maltodextrin product prepared with TERMAMYL 120L Type S *Bacillus stearothermophilus* alpha-amylase showed low turbidity up to 71 hours of storage at 20° C., which is indicative of a clear and stable product.

EXAMPLE 6

In this example, there was produced a liquid maltodextrin having a DE value of about 12.2. The product was produced in accordance with the conditions described in Example 1, except for the following modifications:

a) The starch used was waxy corn starch;
b) The ds of the starch slurry was about 30.7%;
c) The pH of the starch slurry was about 5.8-5.9;
d) No calcium was added;
e) The first dose of alpha-amylase was about 0.01%;
f) The flow rate was about 31,800 liters/hour;
g) The second dose of alpha-amylase was about 0.01 %;
h) The reaction time was about 3.5 hours;
i) The reaction pH was about 5.5 to 5.9;
j) The acid used was 36% hydrochloric acid; and
k) The inactivation pH was about 3.4.

The resultant liquid maltodextrin was characterized by having a DE of about 12.2. The liquid maltodextrin was then refined in accordance with the process of Example 2, except for the following modifications:

a) A rotary vacuum filter available from Eimco was used;
b) Celite's Kenite 3000 fiter aid was used; and
c) Calgon CPG - LF carbon was used.

The resulting refined liquid maltodextrin was concentrated to about 64.2% and stored at 65°C. An evaluation for clarity revealed a percent light transmittance at 600 nm of about 87.6% after 52 days.

EXAMPLE 7

In this example, there was produced a liquid maltodextrin having a DE value of about 10.4. The product was produced in accordance with the conditions described in Example 1, except for the following modifications:

a) The starch used was waxy corn starch;
b) The ds of the starch slurry was about 30.5%;
c) The pH of the starch slurry was about 5.8-5.9;
d) No calcium was added;
e) The first dose of alpha-amylase was about 0.01%;
f) The flow rate was about 33,000 liters/hour;
g) The first liquefact was held at a temperature of about 148°C for about 3 minutes, prior to adding the second dose of alpha - amylase;
h) The second dose of alpha-amylase was about 0.01%;
i) The reaction time was about 4.1 hours;
j) The reaction pH was about 5.4 to 6.3; and
k) The acid used was 36% hydrochloric acid.

The resultant liquid maltodextrin was characterized by having a DE of about 10.4. The liquid maltodextrin was then refined in accordance with the process of Example 2, except for the following modifications:

a) A rotary vacuum filter available from Einco was used;
b) Celite's Kenite 300 filter aid was used;
c) Calgon CPG - LF carbon was used.

The resulting refined liquid maltodextrin having a DE of about 10.4 was concentrated to about 62.7%. A determination for clarity revealed a percent light transmittance at 390 nm of about 79.2% after 28 days.

EXAMPLE 8

In this example there was produced a liquid maltodextrin having a DE value of about 10.8. The product was produced in accordance with the conditions described in Example 1, except for the following modifications:

a) The starch used was waxy corn starch;
b) The ds of the starch slurry was about 31.3%;
c) The pH of the starch slurry was about 5.4 to 6.3;
d) No calcium was added;
e) The first dose of alpha-amylase was about 0.0140;
f) The flow rate was about 33,000 liters/hour;
g) The first liquefact was held at a temperature of about 148°C for about 3 minutes, prior to adding the second dose of alpha - amylase;
h) The second does of alpha - amylase was about 0.01%;
i) The reaction time was about 5.5 hours;
j) The reaction pH was about 5.3 to 6.3; and
k) The acid used was 36% hydrochloric acid.

The resultant liquid maltodextrin having a DE of about 10.8 was then refined in accordance with the process of Example 2, except for the following modifications:

a) A rotary vacuum filter available from Einco was used;
b) Celite's Kenite 300 filter aid was used;
c) Calgon CPG - LF carbon was used.

The resulting refined liquid maltodextrin having a DE of about 10.8 was concentrated to about 64.5%. A determination for clarity revealed a percent light transmittance at 390 nm of about 54.3% after 29 days.

EXAMPLE 9

In this example there was produced a liquid maltodextrin having a DE of about 11.2. The product was produced in accordance with the conditions described in Example 1, except for the following modifications:

a) The starch used was waxy corn starch;
b) The ds of the starch slurry was about 32%;
c) The pH of the starch slurry was about 5.5 to 6.1;
d) No calcium was added;
e) The first dose of alpha-amylase was about 0.015%;
f) The flow rate was about 29,520 liters/hour;
g) The first liquefact was held at a temperature of about 148°C for about 3 minutes; prior to adding the second dose of alpha-amylase;
h) The second dose of alpha-amylase was about 0.01%;
i) The reaction time was about 4.9 hours;
j) The reaction pH was about 5.7 to 5.9; and
k) The acid used was 36% hydrochloric acid.

The resultant liquid maltodextrin having a DE of about 11.2 was then refined in accordance with the process of Example 2, except for the following modifications:

a) A rotary vacuum filter available from Eimco was used;
b) Celite's Kenite 3000 filter aid was used;
c) Calgon CPG - LP carbon was used.

The resultant refined liquid maltodextrin having a DE of about 11.2 was concentrated to about 66.5%. A determination for clarity revealed a percent light transmittance at 390 nm of about 41.4% after 28 days.
The invention has been described with reference to various specific and illustrative embodiments and techniques. However, one skilled in the art will recognize that many variations and modifications may be made while remaining within the scope of the claims.

**Claims**

1. A process for producing liquid maltodextrin having a D.E. of 5 to less than 20 wherein a first liquefact is formed having a D.E. of 0.5 to 5.0, comprising:

a) heating the first liquefact to 120°C to 165°C, and maintaining the first liquefact at said temperature of 120°C to 165°C, for a period of 30 seconds to 10 minutes;
b) adjusting the temperature of the first liquefact to 101° to 115°C., and maintaining the first liquefact at said temperature for up to 15 minutes, in a pressure vessel;
c) contacting the first liquefact with an amount of Bacillus stearothermophilus alpha-amylase to produce a second liquefact; and
d) cooling the temperature of the second liquefact to 93°C. to 100°C., and maintaining the second liquefact at said temperature for a period of time sufficient to produce a second liquefact having a D.E. of 5 to less than 20.

2. A process according to claim 1 for producing liquid maltodextrin having a DE of 5 to less than 20, comprising:

a) mixing a starch with a sufficient amount of water to provide a starch slurry having less than 50% ds;
b) contacting the resultant starch slurry with a first dosage of Bacillus stearothermophilus alpha-amylase, in an amount sufficient to convert or hydrolyze the starch;
c) heating the temperature of the resultant alpha-amylase containing starch slurry to form a first liquefact having a D.E. of 0.5 to 5.0;
d) heating the first liquefact of step 2 (c) to 120°C to 165°C, and maintaining the first liquefact at the temperature of 120°C to 165°C, for a period of 30 seconds to 10 minutes;
e) adjusting the temperature of the first liquefact of step 2 (d), to 101°C to 115°C, and maintaining the first liquefact at the temperature of 101°C to 115°C for a period of up to 15 minutes, in a pressure vessel;
f) contacting the resultant first liquefact from step 2 (e) with a second dosage of Bacillus stearothermophilus alpha-amylase, in an amount sufficient to produce a second liquefact; and

g) cooling the temperature of the second liquefact to 93°C to 100°C, and maintaining the second liquefact at the temperature of 93°C to 100°C for a period of time sufficient to produce a second liquefact having a D.E. of 5 to less than 20.

3. The process according to Claim 2, wherein the starch slurry of step 2(a) ranges from 24 to 40% ds,

4. The process according to Claim 3, wherein the starch slurry of step 2(a) ranges from from 32 to 36% ds.

5. The process according to Claim 2 or 3, further comprising adding 50-100 ppm free calcium to the starch slurry of step 2(a).

6. The process according to Claim 2, wherein the amount of alpha amylase in step 2(b) ranges from 0.01 to 0.09% by weight of the starch on dry basis.

7. The process according to Claim 2, wherein the pH of the alpha-amylase containing starch slurry of step 2(b) is adjusted to 5.0 to 7.0, said pH being maintained throughout the process.

8. The process according to Claim 2, wherein the temperature of the starch slurry of step 2(c) ranges from 80°C to 115°C and the starch slurry is maintained at said temperature for a period of 6 to 15 minutes,

9. The process according to Claim 8, wherein the temperature of the starch slurry of step 2(c) ranges from 107 to 110°C, and the starch slurry is maintained at said temperature for a period of 6 to 15 minutes.

10. The process according to Claim 2, wherein the first liquefact having a D.E. of 0.5 to 5.0, of step 2(c), is cooled prior to step 2(d).

11. The process according to Claim 1 or 2, wherein the temperature of the first liquefact is adjusted to 108° to 110°C., in step 1(b) or 2(e).

12. The process according to Claim 1 or 2, wherein the amount of alpha-amylase in step 1(c) or 2(f) ranges from 0.01 to 0.09% by weight of the starch on dry basis.

13. The process according to Claim 1 or 2, wherein the second liquefact, in step 1(d) or 2(g), is cooled by flash cooling.

14. The process according to Claim 1 or 2, further comprising adjusting the pH of the process to inactivate the alpha-amylase, subsequent to cooling the second liquefact in step 1(d) or 2(g),
preferably wherein the pH is adjusted to 3.4 to 3.7.

15. The process according to Claim 1 or 2, further comprising spray drying or refining the second liquefact having a DE of 5 to less than 20.

16. The process according to Claim 15, wherein the refining is selected from the group consisting of filtration through diatomaceous earth on a vacuum filter, centrifugation, flocculation, flotation, treatment with vegetable carbon and ion exchange resins, and mixtures thereof.


**Patentansprüche**

1. Verfahren zur Herstellung von flüssigem Maltodextrin mit einem Dextrose-Äquivalent (D.E.) von 5 bis weniger als 20, wobei ein erstes Verflüssigungsprodukt gebildet wird, das ein D.E. von 0,5 bis 5,0 hat, umfassend:

a) Erhitzen des ersten Verflüssigungsprodukts auf 120°C bis 165°C und Halten des ersten Verflüssigungsprodukts auf der Temperatur von 120°C bis 165°C für eine Zeitspanne von 30 Sekunden bis 10 Minuten;
b) Einstellen der Temperatur des ersten Verflüssigungsprodukts auf 101 ° bis 115°C und Halten des ersten Verflüssigungsprodukts auf dieser Temperatur bis zu 15 Minuten lang in einem Druckgefäß;
c) Inkontaktbringen des ersten Verflüssigungsprodukts mit einer Menge von Bacillus stearothermophilus Alpha-Amylase zur Herstellung eines zweiten Verflüssigungsprodukts; und

d) Abkühlen der Temperatur des zweiten Verflüssigungsprodukts auf 93°C bis 100°C und Halten des zweiten Verflüssigungsprodukts auf dieser Temperatur für eine Zeitspanne, welche ausreicht, um ein zweites Verflüssigungsprodukt mit einem D.E. von 5 bis weniger als 20 herzustellen.

2. Verfahren nach Anspruch 1 zur Herstellung von flüssigem Maltodextrin mit einem D.E. von 5 bis weniger als 20, umfassend:

a) Vermischen einer Stärke mit einer ausreichenden Wassermenge zur Bereitstellung einer Stärkenaufschlämmung mit weniger als 50% ds (Trockensubstanz);
b) Inkontaktbringen der resultierenden Stärkenaufschlämmung mit einer ersten Dosis Bacillus stearothermophilus Alpha-Amylase in einer ausreichenden Menge zur Umwandlung oder Hydrolysierung der Stärke;
c) Erhitzen der Temperatur der resultierenden Alpha-Amylase enthaltenden Stärkenaufschlämmung zur Bildung eines ersten Verflüssigungsprodukts mit einem D.E. von 0,5 bis 5,0;
d) Erhitzen des ersten Verflüssigungsprodukts von Schritt 2(c) auf 120°C bis 165°C und Halten des ersten Verflüssigungsprodukts auf der Temperatur von 120°C bis 165°C für eine Zeitspanne von 30 Sekunden bis 10 Minuten;
e) Einstellen der Temperatur des ersten Verflüssigungsprodukts von Schritt 2(d) auf 101 °C bis 115°C und Halten des ersten Verflüssigungsprodukts auf der Temperatur von 101 °C bis 115°C für eine Zeitspanne bis zu 15 Minuten in einem Druckgefäß;
f) Inkontaktbringen des resultierenden ersten Verflüssigungsprodukts von Schritt 2(e) mit einer zweiten Dosis Bacillus stearothermophilus Alpha-Amylase in einer ausreichenden Menge zur Herstellung eines zweiten Verflüssigungsprodukts; und
g) Abkühlen der Temperatur des zweiten Verflüssigungsprodukts auf 93°C bis 100°C und Halten des zweiten Verflüssigungsprodukts auf der Temperatur von 93°C bis 100°C für eine ausreichende Zeitspanne zur Herstellung eines zweiten Verflüssigungsprodukts mit einem D.E. von 5 bis weniger als 20.

3. Verfahren nach Anspruch 2, wobei die Stärkenaufschlämmung von Schritt 2(a) im Bereich von 24 bis 40% ds liegt.

4. Verfahren nach Anspruch 3, wobei die Stärkenaufschlämmung von Schritt 2(a) im Bereich von 32 bis 36% ds liegt.

5. Verfahren nach Anspruch 2 oder 3, weiterhin umfassend die Zugabe von 50-100 ppm freien Calciums zur Stärkenaufschlämmung von Schritt 2(a).

6. Verfahren nach Anspruch 2, wobei die Menge der Alpha-Amylase in Schritt 2(b) im Bereich von 0,01 bis 0,09 Gew.-% der Stärke auf Trockenbasis liegt.

7. Verfahren nach Anspruch 2, wobei der pH der Alpha-Amylase enthaltenden Stärkenaufschlämmung von Schritt 2 (b) auf 5,0 bis 7,0 eingestellt wird, wobei der pH während des Verfahrens aufrechterhalten wird.

8. Verfahren nach Anspruch 2, wobei die Temperatur der Stärkenaufschlämmung von Schritt 2(c) im Bereich von 80°C bis 115°C liegt und die Stärkenaufschlämmung auf der Temperatur für eine Zeitspanne von 6 bis 15 Minuten gehalten wird.

9. Verfahren nach Anspruch 8, wobei die Temperatur der Stärkenaufschlämmung von Schritt 2 (c) im Bereich von 107 bis 110°C liegt und die Stärkenaufschlämmung auf dieser Temperatur für eine Zeitspanne von 6 bis 15 Minuten gehalten wird.

10. Verfahren nach Anspruch 2, wobei das erste Verflüssigungsprodukt mit einem D.E. von 0,5 bis 5,0 von Schritt 2(c) vor dem Schritt 2(d) abgekühlt wird.

11. Verfahren nach Anspruch 1 oder 2, wobei die Temperatur des ersten Verflüssigungsprodukts in Schritt 1 (b) oder 2(e) auf 108° bis 110°C eingestellt wird.

12. Verfahren nach Anspruch 1 oder 2, wobei die Menge der Alpha-Amylase in Schritt 1 (c) oder 2(f) im Bereich von 0,01 bis 0,09 Gew.-% der Stärke auf Trockenbasis liegt.

13. Verfahren nach Anspruch 1 oder 2, wobei das zweite Verflüssigungsprodukt in Schritt 1 (d) oder 2(g) durch Blitzkühlung abgekühlt wird.

**14.** Verfahren nach Anspruch 1 oder 2, weiter umfassend das Einstellen des pH des Verfahrens zur Inaktivierung der Alpha-Amylase nach dem Abkühlen des zweiten Verflüssigungsprodukts in Schritt 1 (d) oder 2(g), wobei der pH bevorzugt auf 3,4 bis 3,7 eingestellt wird.

**15.** Verfahren nach Anspruch 1 oder 2, weiter umfassend das Sprühtrocken oder Raffinieren des zweiten Verflüssigungsprodukts mit einem D.E. von 5 bis weniger als 20.

**16.** Verfahren nach Anspruch 15, wobei das Raffinieren aus der Gruppe ausgewählt ist, die aus Filtrieren durch Diatomeenerde auf einem Vakuumfilter, Zentrifugieren, Flokkulation, Flotation, Behandlung mit Pflanzenkohle und Ionenaustauschharzen sowie Gemischen davon besteht.

**Revendications**

**1.** Un procédé de production de maltodextrine liquide ayant un E.D. situé entre 5 et moins de 20, dans lequel un premier liquefact ayant un E.D. situé entre 0,5 et 5,0 est formé, se composant des opérations suivantes :

a) chauffer le premier liquefact pour l'amener à une température située entre 120 °C et 165 °C et maintenir le premier liquefact à cette température pendant une période allant de 30 secondes à 10 minutes ;
b) amener le premier liquefact à une température située entre 101 °C et 115 °C et maintenir le premier liquefact à cette température pendant jusqu'à 15 minutes, dans un récipient sous pression ;
c) mettre le premier liquefact en contact avec une quantité de Bacillus stearothermophilus alpha-amylase afin d'obtenir un deuxième liquefact ; et
d) abaisser la température du deuxième liquefact pour l'amener entre 93 °C et 100 °C et maintenir le deuxième liquefact à cette température pendant une durée suffisante pour obtenir un deuxième liquefact ayant un E.D. situé entre 5 et moins de 20.

**2.** Un procédé selon la revendication 1, permettant de produire une maltodextrine liquide ayant un E.D. situé entre 5 et moins de 20, se composant des opérations suivantes :

a) mélanger un amidon avec une quantité d'eau suffisante pour obtenir une suspension d'amidon ayant moins de 50 % de matière sèche ;
b) mettre la suspension d'amidon ainsi obtenue en contact avec une première dose d'alpha-amylase de Bacillus stearothermophilus dans une quantité suffisante pour convertir ou hydrolyser l'amidon ;
c) chauffer la température de la suspension d'amidon contenant l'alpha-amylase ainsi obtenue afin d'obtenir un premier liquefact ayant un E.D. situé entre 0,5 et 5,0 ;
d) chauffer le premier liquefact de l'étape 2(c) pour l'amener à une température située entre 120 °C et 165 °C et maintenir le premier liquefact à cette température pendant une période allant de 30 secondes à 10 minutes ;
e) amener la température du premier liquefact de l'étape 2(d) à une température située entre 101 °C et 115 °C et maintenir le premier liquefact à cette température pendant une période allant jusqu'à 15 minutes, dans un récipient sous pression ;
f) mettre le premier liquefact obtenu à l'étape 2(e) en contact avec une deuxième dose d'alpha-amylase de Bacillus stearothermophilus, dans une quantité suffisante pour produire un deuxième liquefact ; et
g) abaisser la température du deuxième liquefact pour l'amener entre 93 °C et 100 °C et maintenir le deuxième liquefact à cette température pendant une période suffisante pour produire un deuxième liquefact ayant un E.D. situé entre 5 et moins de 20.

**3.** Le procédé de la revendication 2, dans lequel la suspension d'amidon de l'étape 2(a) possède entre 24 et 40 % de matière sèche.

**4.** Le procédé de la revendication 3, dans lequel la suspension d'amidon de l'étape 2(a) possède entre 32 et 36 % de matière sèche.

**5.** Le procédé des revendications 2 ou 3, supposant également d'ajouter de 50 à 100 ppm de calcium libre à la suspension d'amidon de l'étape 2(a).

**6.** Le procédé de la revendication 2, dans lequel la quantité d'alpha-amylase de l'étape 2(b) se situe entre 0,01 et 0,09 % par poids de l'amidon sur une base sèche.

**7.** Le procédé de la revendication 2, dans lequel le pH de la suspension d'amidon contenant l'alpha-amylase de l'étape 2(b) est amené entre 5,0 et 7,0, ce pH étant maintenu pendant tout le processus.

**8.** Le procédé de la revendication 2, dans lequel la température de la suspension d'amidon de l'étape 2(c) se situe entre 80 °C et 115 °C et la suspension d'amidon est maintenue à cette température pendant une période allant de 6 à 15 minutes.

**9.** Le procédé de la revendication 8, dans lequel la température de la suspension d'amidon de l'étape 2(c) se situe entre 107 °C et 110 °C et la suspension d'amidon est maintenue à cette température pendant une période allant de 6 à 15 minutes.

**10.** Le procédé de la revendication 2, dans lequel le premier liquefact ayant un E.D. situé entre 0,5 et 5,0 de l'étape 2 (c) est refroidi avant l'étape 2(d).

**11.** Le procédé des revendications 1 ou 2, dans lequel la température du premier liquefact est amenée entre 108 °C et 110 °C à l'étape 1 (b) ou 2(e).

**12.** Le procédé des revendications 1 ou 2, dans lequel la quantité d'alpha-amylase utilisée à l'étape 1 (c) ou 2(f) se situe entre 0,01 et 0,09 % par poids de l'amidon sur une base sèche.

**13.** Le procédé des revendications 1 ou 2, dans lequel le deuxième liquefact, à l'étape 1 (d) ou 2(g), est refroidi par refroidissement instantané.

**14.** Le procédé des revendications 1 ou 2, supposant également d'ajuster le pH du procédé pour inactiver l'alpha-amylase après le refroidissement du deuxième liquefact à l'étape 1 (d) ou 2(g),
dans lequel le pH est de préférence amené entre 3,4 et 3,7.

**15.** Le procédé des revendications 1 ou 2, supposant également de sécher par pulvérisation ou d'affiner le deuxième liquefact ayant un E.D. situé entre 5 et moins de 20.

**16.** Le procédé de la revendication 15, dans lequel la technique d'affinage est sélectionnée parmi les suivantes : filtration par terres à diatomées sur un filtre sous vide, centrifugation, floculation, flottation, traitement au charbon végétal et résines échangeuses d'ions, et leurs mélanges.

**EP 1 811 863 B1**

## REFERENCES CITED IN THE DESCRIPTION

### Patent documents cited in the description

- US 3849194 A
- US 3853706 A
- US 4284722 A
- US 4447532 A
- US 5612202 A